# EUROPEAN PATENT SPECIFICATION

(11) **EP 3 111 920 B1**
(45) Date of publication and mention of the grant of the patent: **28.08.2019**
(21) Application number: 15754782.9
(22) Date of filing: 20.02.2015
(51) Int. Cl.: A61K 8/49, A61K 8/46, A61K 8/73, A61K 31/635, A61K 31/722, A61P 17/14, A61Q 7/00

(54) **COMPOSITION FOR PROMOTING HAIR GROWTH AND/OR HAIR RESTORATION CONTAINING SULFA AGENT AND CHITOSAN AGENT**
ZUSAMMENSETZUNG ZUR FÖRDERUNG DES HAARWACHSTUMS UND/ODER DER HAARERNEUERUNG MIT EINEM SULFAMITTEL UND CHITOSANMITTEL
COMPOSITION POUR FAVORISER LA POUSSE ET/OU LA CROISSANCE DES CHEVEUX CONTENANT UN SULFAMIDE ET UN CHITOSANE

(30) Priority: 27.02.2014 JP 2014036449
(43) Date of publication of application: 04.01.2017
(73) Proprietor: Greenever, Kitasaku-gun, Nagano 389-0115 (JP)
(72) Inventor: SONOO, Yoshiko, Kitasaku-gun Nagano 389-0115 (JP)
(74) Representative: Hoffmann Eitle
(86) International application number: PCT/JP2015/054732
(87) International publication number: WO 2015/129564

(56) References cited:
- DE-A1- 2 322 712
- FR-A1- 2 860 431
- JP-A- H0 656 634
- JP-A- H0 753 334
- JP-A- H0 820 514
- JP-A- S5 946 223
- JP-A- H02 273 610
- JP-A- H08 127 518
- JP-A- H09 183 712
- JP-A- H09 241 134
- JP-A- 2002 516 846
- JP-A- 2002 525 305
- JP-A- 2008 069 116
- JP-A- 2011 529 938
- JP-A- 2012 523 449
- DATABASE GNPD [Online] MINTEL; 1 June 2009 (2009-06-01), "Fortifying Repair Mousse", XP002773720, Database accession no. 1113031
- DATABASE GNPD [Online] MINTEL; 1 August 2007 (2007-08-01), "Anti-Hair Loss Treatment", XP002773721, Database accession no. 761005

## Description

### TECHNICAL FIELD

The present invention relates to a composition for promoting hair restoration and/or hair growth comprising a sulfa agent and a chitosan agent. In addition, this also provides a kit comprising the composition for promoting hair restoration and/or hair growth of the present invention and a process for applying the composition for promoting hair restoration and/or hair growth of the present invention.

### BACKGROUND ART

Thin hair or alopecia can be caused by various factors such as heredity, aging, inflammation, stress, side effects by a drug, etc. Alopecia largely affects the impression on the appearance, and there is a large social impact.

There are various products for prevention or reduction of alopecia or promotion of hair restoration and hair growth, and for example, there are products that state promotion of blood circulation, strengthen of function of a hair matrix cell, moisture retention of scalp and inhibition of male hormone function. For the male type alopecia, minoxidil, finasteride, carpronium chloride, t-flavanone, adenosine, cytopurine pentadecane, etc., have been used. However, a hair growth promoting effect by these components is not sufficient, and some of which have side effects such as sexual dysfunction, etc. It has been desired to develop a hair growth promoting composition having excellent effect without any side effects.

On the other hand, a sulfa agent has been used for a long time to prevent and treat an infection in the wound. The sulfa agent is a general term of a synthetic compound having a sulfonamide group. The sulfa agent inhibits by antagonizing p-aminobenzoic acid which is a substrate of an essential enzyme, dihydropteroate synthase in the folic acid synthetic pathway. As a result, the sulfa agent inhibits folic acid synthesis in bacteria and DNA/RNA synthesis which requires folic acid to show an antibacterial effect.

Also, the sulfa agent prevents formation of bicarbonate ions by inhibiting carbonic anhydrase and shows a function of lowering a blood pH. According to this matter, a pH at the wound part is lowered than the growth optimum pH of bacteria, whereby it shows an antibacterial action. Furthermore, JP 2002-516846 discloses a method for treating alopecia or promoting hair growth with a small molecule sulfonamide.

A chitosan agent has been well used in wound treatment. When the chitosan agent containing D-glucosamine polymer as a main component is applied to the wound part, it induces fibroblast, promotes formation of granulation, and promotes wound healing. Furthermore, JP 8-20514 discloses a hair growth agent characterized by containing chitosan.

It has been known a medical cream agent containing a sulfa agent and a chitosan agent, for recovering a skin and for treating bacterial skin infection (Patent document 1). However, it has never been known that the above-mentioned medical cream agent has a hair restoration or hair growth function.

### PRIOR ART DOCUMENT

### PATENT DOCUMENT

Patent document 1: WO2010-119369A

### SUMMARY OF THE INVENTION

### PROBLEMS TO BE SOLVED BY THE INVENTION

An object of the present invention is to provide a novel composition for promoting hair restoration and/or hair growth.

### MEANS TO SOLVE THE PROBLEMS

The present inventor has earnestly studied and as a result, found that a composition comprising a sulfa agent and a chitosan agent has a hair restoration and hair growth effect, whereby the present invention has been accomplished.

Described herein is:
[1] A composition for promoting hair restoration and/or hair growth comprising a sulfa agent and a chitosan agent.
[2] The composition for promoting hair restoration and/or hair growth described in [1], wherein the sulfa agent is selected from the group consisting of sulfamonomethoxine, acetyl sulfamethoxazole, salazosulfapyridine, sulfadiazine, silver sulfadiazine, sulfadimethoxine, sulfathiazole, sulfaphenazole, sulfamethoxazole, sulfamethoxypyridazine, sulfamethopyrazine, sulfametomidine, sulfamethizole, sulfamerazine, sulfisoxazole, sulfisomidine, sulfisomidine sodium, homosulfamine and a derivative thereof.
[3] The composition for promoting hair restoration and/or hair growth described in [1] or [2], wherein the sulfa agent is sulfamonomethoxine or a derivative thereof.
[4] The composition for promoting hair restoration and/or hair growth described in any one of [1] to [3], wherein the chitosan agent is a mixture of a high molecular weight chitosan and a low molecular weight chitosan.
[5] The composition for promoting hair restoration and/or hair growth described in any one of [1] to [4], wherein the chitosan agent comprises chitin.
[6] The composition for promoting hair restoration and/or hair growth described in any one of [1] to [5], wherein the sulfa agent and the chitosan agent are mixed at a weight ratio of 20:1 to 1:20.
[7] The composition for promoting hair growth and/or hair restoration described in any one of [1] to [6], wherein the sulfa agent and the chitosan agent are mixed at a weight ratio of 5:1 to 1:5.
[8] The composition for promoting hair restoration and/or hair growth described in any one of [1] to [7], wherein a dosage form is powder or a liquid formulation.
[9] The composition for promoting hair restoration and/or hair growth described in any one of [1] to [8], wherein the hair restoration and/or hair growth is hair restoration and/or hair growth by alopecia selected from the group consisting of wound-derived alopecia, senile alopecia, stress-derived alopecia, male type alopecia, scarring alopecia, drug-derived alopecia and postpartum alopecia.
[10] The composition for promoting hair restoration and/or hair growth described in any one of [1] to [9], wherein an object is birds or mammals.
[11] The composition for promoting hair restoration and/or hair growth described in [10], wherein the object is mammals selected from the group consisting of dogs, cats, horses, sheep, cattle and human.
[12] A process for preparing the composition for promoting hair restoration and/or hair growth described in any one of [1] to [11], which comprises a step of mixing a sulfa agent and a chitosan agent.
[13] A sulfa agent for applying separately from or simultaneously with a chitosan agent to an area of an object at which hair restoration and/or hair growth is required.
[14] A chitosan agent for applying separately from or simultaneously with a sulfa agent to an area of an object at which hair restoration and/or hair growth is required,.
[15] A kit comprising a sulfa agent, a chitosan agent and optionally a dissipation container.
[16] The kit described in [15], wherein the dissipation container has a dropper shape or a spray type.
[17] A method for promoting hair restoration and/or hair growth of an object, which comprises a step of:
   a) a step of applying a sulfa agent and a chitosan agent separately or simultaneously to an area of an object at which hair restoration and/or hair growth is required.
[18] The method described in [17], wherein the sulfa agent is selected from the group consisting of sulfamonomethoxine, acetyl sulfamethoxazole, salazosulfapyridine, sulfadiazine, silver sulfadiazine, sulfadimethoxine, sulfathiazole, sulfaphenazole, sulfamethoxazole, sulfamethoxypyridazine, sulfamethopyrazine, sulfametomidine, sulfamethizole, sulfamerazine, sulfisoxazole, sulfisomidine, sulfisomidine sodium, homosulfamine and a derivative thereof.
[19] The method described in [17] or [18], wherein the sulfa agent is sulfamonomethoxine or a derivative thereof.
[20] The method described in any one of [17] to [19], wherein the chitosan agent is a mixture of a high molecular weight chitosan and a low molecular weight chitosan.
[21] The method described in any one of [17] to [20], wherein the chitosan agent comprises chitin.
[22] The method described in any one of [17] to [21], wherein the sulfa agent and the chitosan agent to be applied are at a weight ratio of 20:1 to 1:20.
[23] The method described in any one of [17] to [22], wherein the sulfa agent and the chitosan agent to be applied are at a weight ratio of 5:1 to 1:5.
[24] The method described in any one of [17] to [23], wherein the sulfa agent and the chitosan agent to be applied are mixed in advance.
[25] The method described in any one of [17] to [24], wherein a dosage forms of the sulfa agent and the chitosan agent to be applied is powder or a liquid formulation.
[26] The method described in any one of [17] to [25], wherein the application is carried out by using a dissipation container.
[27] The method described in any one of [17] to [26], wherein the object is suffered from alopecia selected from the group consisting of wound-derived alopecia, senile alopecia, stress-derived alopecia, male type alopecia, scarring alopecia, drug-derived alopecia and postpartum alopecia.
[28] The method described in any one of [17] to [27], wherein the object is birds or mammals.
[29] The method described in [28], wherein the object is mammals selected from the group consisting of dogs, cats, horses, sheep, cattle and human.
[30] A kit comprising a sulfa agent, a chitosan agent and optionally a dissipation container to be used for the method described in [17] to [29].

The present invention is defined by the appended claims.

### EFFECTS OF THE INVENTION

According to the present invention, a composition for promoting hair restoration and/or hair growth which is different from the conventional hair growing agent is provided.

### BRIEF DESCRIPTION OF THE DRAWINGS

Fig. 1 shows progress of Case 1 by the composition for promoting hair restoration and/or hair growth of the present invention.
Fig. 2 shows progress of Case 2 by the composition for promoting hair restoration and/or hair growth of the present invention.
Fig. 3 shows progress of Case 3 by the composition for promoting hair restoration and/or hair growth of the present invention.
Fig. 4 shows progress of Case 4 by the composition for promoting hair restoration and/or hair growth of the present invention.
Fig. 5 shows progress of Case 5 by the composition for promoting hair restoration and/or hair growth of the present invention.

### EMBODIMENT TO CARRY OUT THE INVENTION

The present invention is to provide a composition for promoting hair restoration and/or hair growth comprising a sulfa agent and a chitosan agent.

The composition for promoting hair growth and/or hair restoration of the present invention comprises a sulfa agent. The sulfa agent in the present invention means any synthetic compound having a sulfonamide group which can be expected to be used for a medicine. More specifically, it may be mentioned sulfamonomethoxine, acetyl sulfamethoxazole, salazosulfapyridine, sulfadiazine, silver sulfadiazine, sulfadimethoxine, sulfathiazole, sulfaphenazole, sulfamethoxazole, sulfamethoxypyridazine, sulfamethopyrazine, sulfametomidine, sulfamethizole, sulfamerazine, sulfisoxazole, sulfisomidine, sulfisomidine sodium and homosulfamine but is not limited thereto. These sulfa agents may be a derivative, for example, in the form of a salt such as a silver salt, etc. In a specific embodiment, the sulfa agent is sulfamonomethoxine or sulfadimethoxine. Also, in another specific embodiment, the sulfa agent is sulfamonomethoxine.

The composition for promoting hair restoration and/or hair growth of the present invention comprises a chitosan agent. In the present invention, the chitosan agent means a mixture comprising chitosan which is a linear type polymer of D-glucosamine as a main component. A polymerization degree and a molecular weight of the chitosan agent are not particularly limited. In a specific embodiment, the chitosan agent of the present invention is a mixture of a high molecular weight chitosan (a molecular weight of 10,000 or higher) and a low molecular weight chitosan (a molecular weight of less than 10,000). A mixing ratio of the high molecular weight chitosan and the low molecular weight chitosan is not limited and, for example, a mixing ratio of 1:9 to 9:1 in a weight ratio may be mentioned.

The chitosan agent in the present invention may further comprise chitin. The chitin means a linear type polymer of N-acetylglucosamine and glucosamine. A polymerization degree of the chitin is not particularly limited, and it may be an oligosaccharide (polymerization degree: 3 to 10). When the chitin is contained in the chitosan agent of the present invention, a content of the chitin is preferably 15% or less in a weight ratio.

In a preferred embodiment, the composition for promoting hair restoration and/or hair growth of the present invention is a chitosan agent comprising sulfamonomethoxine and chitin. In a more preferred embodiment, a ratio of the high molecular weight chitosan: the low molecular weight chitosan: the chitin oligosaccharide in the chitosan agent comprising the chitin is 39:59:2 in a weight ratio.

In the composition for promoting hair restoration and/or hair growth of the present invention, a mixing ratio of the sulfa agent and the chitosan agent of the present invention is not particularly limited, and it may be freely set depending on a kind and a degree of alopecia which is an object for hair restoration and/or hair growth. In an embodiment, the sulfa agent and the chitosan agent of the present invention are mixed at a weight ratio of 20:1 to 1:20. In a preferred embodiment, the sulfa agent and the chitosan agent are mixed at a weight ratio of 5:1 to 1:5.

A dosage form of the composition for promoting hair restoration and/or hair growth of the present invention is not particularly limited so long as it can be applied to an area of an object for hair growth, and may be mentioned a liquid formulation, powder, a gel preparation, cream, etc. When it is also applied to an area with a wide range, the dosage form is preferably a liquid formulation. Also, in another embodiment, to the wound-derived alopecia, it may be used in a powder form so as to treat wound as well. In the present specification, the liquid formulation is not necessary a solution in which the sulfa agent and the chitosan agent are completely dissolved, and may be a state of suspension. The solvent for the solution is not particularly limited, and may be mentioned water, physiological saline, an alcohol, corn oil, etc.

The composition for promoting hair restoration and/or hair growth of the present invention may comprise an optional pharmaceutically acceptable excipient, carrier, diluent, etc., so long as the effect of the present invention is not impaired. Specific examples of the excipient may be mentioned lactose, white sugar, corn starch, etc., but is not limited thereto.

The composition of the present invention can be provided as, for example, medicine, a quasi-drug or a cosmetic.

In the present invention, the terms of "promoting hair restoration and/or hair growth" widely include, in the technical field to which the present invention belongs, a hair amount increasing phenomenon which is the so-called hair growth or hair restoration. For example, promotion of hair restoration includes to regenerate hair cells by causing cell division of a hair matrix cell by stimulating a hair root at the telogen phase in a hair cycle or to generate pores at the area at which no pore had been present until now, but it is not limited thereto. In addition, for example, promotion of hair growth includes to suppress a hair root at the anagen phase in a hair cycle from transferring to the catagen phase or the telogen phase, but it is not limited thereto. The composition of the present invention can cause differentiation from a hair root stem cell to a hair matrix cell, or cell division of a hair matrix cell by lowering a pH around a hair root stem cell by the sulfa agent. In addition, in Case 1, Case 3 and Case 4 explained in detail in Examples, a hair growth effect can be more recognized at the wound skin than the normal skin, so that differentiation of a hair matrix cell can be caused by a synergistic effect of stimulation by wound and a local oxidation action by the sulfa agent.

The composition for promoting hair restoration and/or hair growth of the present invention can be applied for any alopecia. The alopecia more specifically includes wound-derived alopecia, senile alopecia, stress-derived alopecia, male type alopecia, scarring alopecia, drug alopecia and postpartum alopecia. In connection with this, wound-derived alopecia also includes the state in which the wound is in progress actually.

An object to which the composition for promoting hair restoration and/or hair growth of the present invention is applied may be any animal having hair. The object may be more specifically birds, mammals, etc., but is not limited thereto. In an embodiment, the object is mammals, and may be mentioned, for example, dogs, cats, horses, sheep, cattle and human.

A method for applying the composition for promoting hair restoration and/or hair growth is described. In an embodiment, the composition for promoting hair restoration and/or hair growth of the present invention is directly coated to the area to be applied. Also, in another embodiment, the composition for promoting hair restoration and/or hair growth of the present invention is applied by using a dissipation container without directly touching to the area to be applied by an operator. The dissipation container to be used in this case may be any container which can spread the composition for promoting hair restoration and/or hair growth of the present invention, and it may be used, for example, a spuit container for eye dropping or a spray container.

Also described herein is a process for preparing the composition for promoting hair restoration and/or hair growth of the present invention which comprises mixing a sulfa agent, a chitosan agent and an excipient, if necessary.

Starting materials to be used for the preparation process of the present invention may be preferably the respective compounds, etc., exemplified in the composition for promoting hair restoration and/or hair growth of the present invention.

A method for obtaining the respective starting materials to be used in the preparation process is not particularly limited. With regard to the chitosan agent, it may be obtained by deacetylating chitin by the conventionally known method.

A mixing method in the preparation process is not particularly limited so long as the sulfa agent and the chitosan agent are uniformly mixed. In an embodiment, the chitosan agent having light specific gravity is firstly charged in a mixing apparatus, the sulfa agent having heavy specific gravity is then charged therein and mixed by stirring. According to this embodiment, the materials are rapidly and uniformly mixed.

Also described herein is the provision of a sulfa agent in a powder form for applying it separately from or simultaneously with a chitosan agent to an area at which hair restoration and/or hair growth of an object is desired .

The sulfa agent may be more specifically mentioned the sulfa agent exemplified in the composition for promoting hair restoration and/or hair growth of the present invention.

Also described herein is the provision of a chitosan agent in a powder form for applying it separately from or simultaneously with a sulfa agent to an area at which hair restoration and/or hair growth of an object is desired.

The chitosan agent in a powder form may be more specifically mentioned the chitosan agent exemplified in the composition for promoting hair restoration and/or hair growth of the present invention.

The present invention is also to provide a kit for promoting hair restoration and/or hair growth comprising a sulfa agent, a chitosan agent and occasionally a dissipation container.

In an embodiment, the kit for promoting hair restoration and/or hair growth of the present invention contains a dissipation container. The dissipation container may be any container which can spray the composition for promoting hair restoration and/or hair growth of the present invention and is, for example, a spuit container for eye dropping or a spray container.

The sulfa agent and the chitosan agent suitable in the kit for promoting hair restoration and/or hair growth of the present invention may be mentioned the respective compound, etc., exemplified in the composition for promoting hair restoration and/or hair growth of the present invention.

Also described herein is a method for promoting hair restoration and/or hair growth of an object, which comprises a step of:
a) a step for applying the sulfa agent and the chitosan agent separately or simultaneously to an area of the object at which hair restoration and/or hair growth is desired.

According to the method, hair restoration and/or hair growth can be promoted simply and easily, and effectively than the conventional hair growth method.

In the method described herein, the sulfa agent and the chitosan agent may be applied separately, or may be applied simultaneously. When they are applied simultaneously, it is preferred since the composition for treatment of the present invention can be used.

The sulfa agent and the chitosan agent to be applied by the method of the present invention may be mentioned the respective compounds exemplified in the composition for treatment of the present invention.

In the method described herein, in an embodiment, the sulfa agent and the chitosan agent can be directly applied. Also, in another embodiment, a dissipation container may be used when it is applied. The dissipation container may be any container which can spray the composition for promoting hair restoration and/or hair growth of the present invention and may be mentioned, for example, a spuit container for eye drops or a spray container.

In the method described herein, at the time of applying the sulfa agent and the chitosan agent or after applying the same, the area to be applied may be subjected to patting. Strength and frequency of the patting can be optionally controlled depending on the condition of the area to be applied.

Alopecia in the present invention is alopecia to which the composition for promoting hair restoration and/or hair growth of the present invention can be applied.

The object in the present invention is an object to which the composition for promoting hair restoration and/or hair growth of the present invention can be applied. It is more preferably optional animals excluding human, further preferably mammals excluding human.

Application doses of the sulfa agent and the chitosan agent in the present invention are not limited so long as these can accomplish the effect of the present invention. The application dose may be sufficient with a dose, for example, in which the composition for promoting hair restoration and/or hair growth of the present invention is touched to the area at which hair restoration and/or hair growth is desired.

A number of the application and frequency in the method of the present invention are not limited so long as the effect of the present invention can be accomplished. In an embodiment, frequency to be applied is 1, 2, 3, 4 or 5 times in a day, or once in 2 days, once in 3 days, once in 4 days, once in 5 days, once in 6 days or once in a week.

### EXAMPLES

Next, the present invention is explained in more detail by referring to Examples, but the present invention is not limited by the following Examples as long as it does not beyond the gist of the invention.

### [Preparation of drug combination powder and drug combination suspension containing sulfa agent and chitosan agent]

As the sulfa agent, Daimeton Powder 20% (Meiji Seika Pharma, Japan) was used. Daimeton Powder is powder containing 20 g of sulfamonomethoxine in 100 g thereof.

As the chitosan agent, powder (Mori Kenshou, Japan) in which high molecular weight chitosan (Flonac H, Nippon Suisan Kaisha, Ltd., Japan), low molecular weight chitosan (Flonac C-100M, Nippon Suisan Kaisha, Ltd., Japan) and chitin oligosaccharide had been mixed with a weight ratio of 39:59:2 was used. Incidentally, a purification degree (a deacetylation degree) of high molecular weight chitosan and the low molecular weight chitosan is both about 90%, and chitin which had not been deacetylated is considered to be contained with about 10%.

A drug combination powder containing the sulfa agent and the chitosan agent was prepared as follows:

| | |
|---|---|
| Drug combination powder | volume ratio 1:1 10 ml |
| Daimeton Powder | 3.5 g (sulfamonomethoxine: 0.7 g) |
| Chitosan agent | 1.5 g |

Into 10 ml of an eye drop bottle were charged a chitosan agent and Daimeton Powder successively, and they were well mixed by shaking.

A combined liquid formulation containing the sulfa agent and the chitosan agent was prepared by suspending the above-mentioned drug combination powder in a tap water with 1% (weight/volume).

### [Case 1: Treatment by drug combination powder in cat]

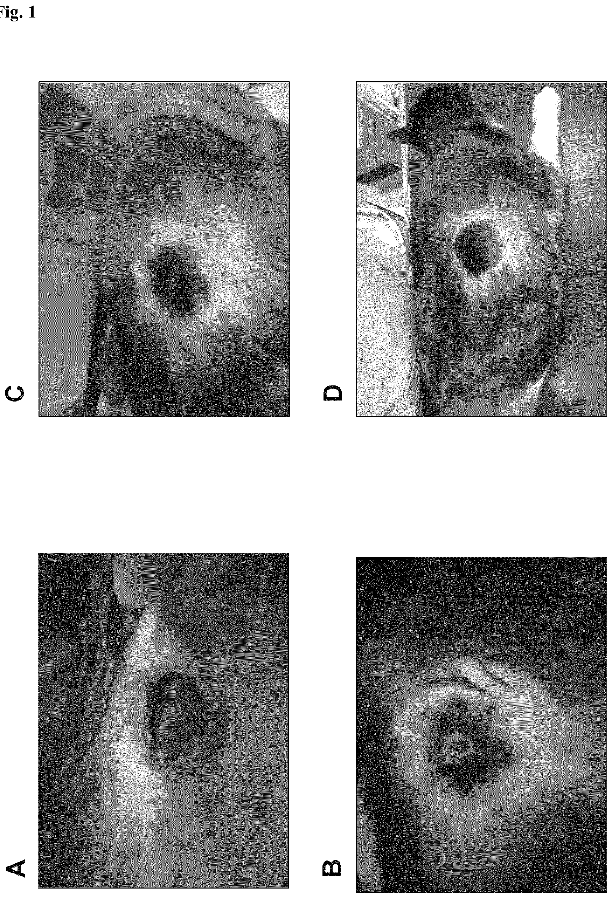

A female calico cat subjected to contraception with estimated 15-years-old that had been skin defected by a wound reached to the subcutaneous tissue was treated by drug combination powder. The drug combination powder was applied once a day so that it covered the wound part. The state before the treatment is shown in Fig. 1A. The part around the wound was shaved so that the treatment became easy. After 20 days were lapsed from starting the treatment, when the wound part was regenerated and healed, colored hair restoration was admitted at the regenerated part, and the hair restoration rate was faster in the wound healing part than that of the shaved part (Fig. 1B). When 27 days were lapsed from starting the treatment, all the wound part was covered by an epithelium and hair with a tortoiseshell pattern was admitted. The treatment was further continued and 17 days were lapsed, the wound part was completely healed, fur of the original wound part was not differentiated from fur of the usual part (Fig. 1D).

### [Treatment example 2: Treatment by combined liquid formulation in dog]

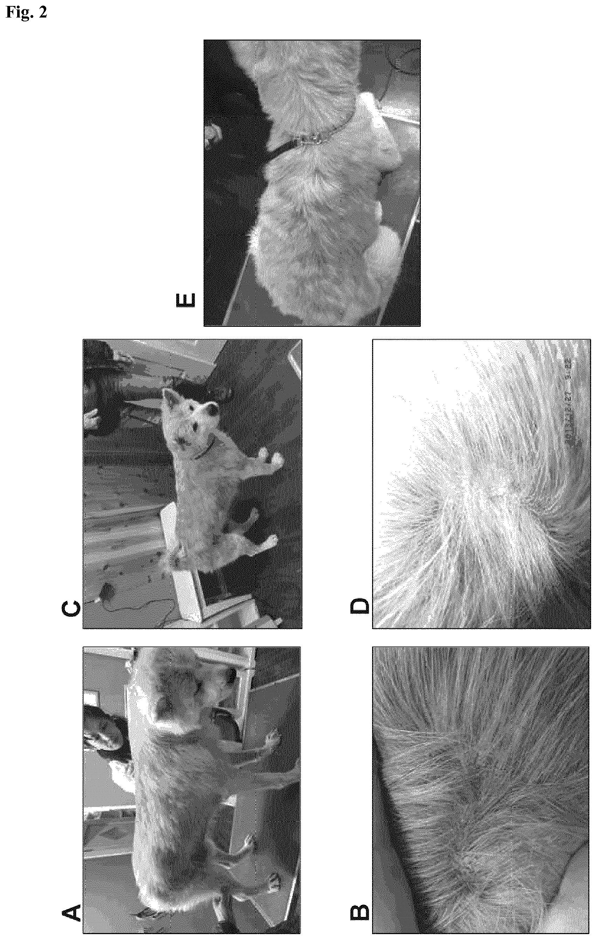

A male dog subjected to contraception with estimated 6-years-old that had been considered to be hair lost caused by both of seborrheic dermatitis and hypothyroidism was treated by a combined liquid formulation. The combined liquid formulation was coated to the whole body while patting once a day. In addition, the dog was treated with shampoo once a week. The states before the treatment are shown in Figs. 2A and B. Enrichment of the hair of the whole body was low (Fig. 2A), undercoat was lost and stiff hair alone was present (Fig. 2B). After 39 days were lapsed from starting the treatment, a hair amount of the whole body was increased (Fig. 2C). Also, undercoat has come to be recognized (Fig. 2D). The treatment was further continued and 24 days were lapsed, dermatitis was generated, but the amount of hair was maintained in sufficient quantity (Fig. 2E).

### [Treatment example 3: Treatment by combined liquid formulation of alopecia areata in dog]

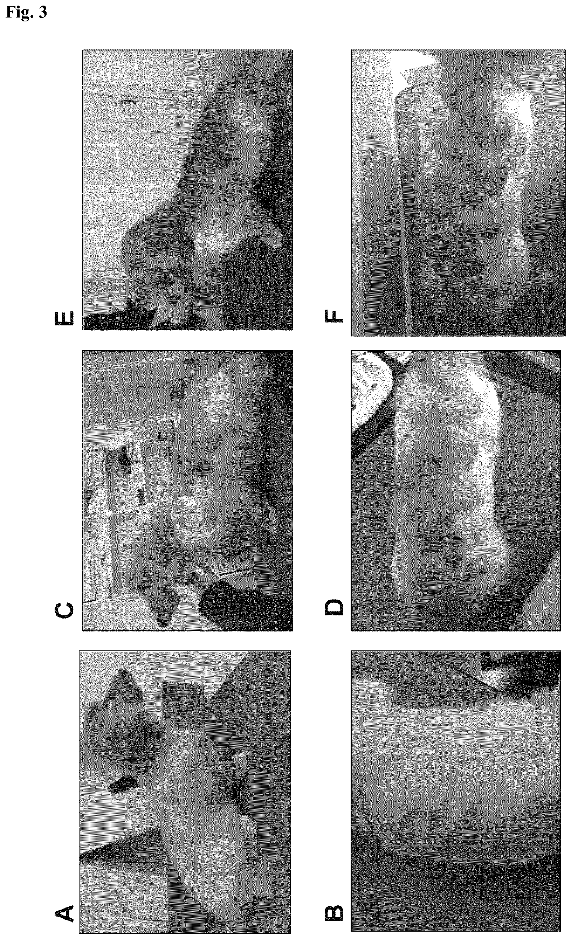

A female dog subjected to contraception with estimated 9-years-old that caused alopecia areata accompanied by flare due to allergic dermatitis was treated by a combined liquid formulation. The combined liquid formulation was coated to the whole body while patting once in 5 days. In addition, the dog was treated with shampoo once in 10 days. The states before the treatment are shown in Fig. 3A and B. When trimming was applied to the dog and the hair of the whole body was cut dermatitis flare were observed at the plural parts of the back, and the flare part was observed to be hair-lost in a circular (Fig. 3B). After 68 days were lapsed from starting the treatment, flare was lost and hair growth was observed (Figs. 3C and D). Interestingly, darker and longer hair was observed at the part where the flare was present than the part that had been cut before the treatment (Fig. 3D). Further 23 days were lapsed, hair of the whole body was so enriched that the part where the flare had been present could not be recognized (Figs. 3E and F).

### [Treatment example 4: Treatment by combined agent of sewing part after eyeball removing operation in cat]

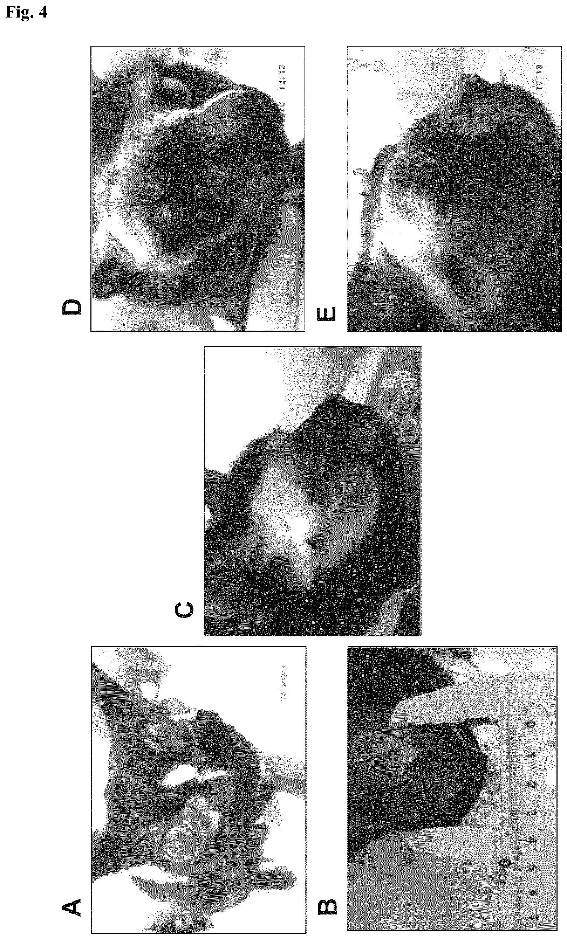

A female cat subjected to contraception with estimated 15-years-old with orbital abscess was subjected to eyeball removing operation. The state before the treatment is shown in Fig. 4A. By shaving hair, severe inflammation was observed, in particular, at upper eyelid (Fig. 4B). In the eyeball removing operation, drug combination powder was sprinkled to the orbit after removing the eyeball, and after inserting lipoma possessed at the abdomen into the orbit, and the eye was sewed. After the removing operation, the drug combination powder was coated around the sewed part once a day. After 28 days were lapsed from starting the treatment, hair growing with a faster rate than the usual hair growth rate could be observed at the hair shaved area around the sewed part (Fig. 4C). The treatment by the drug combination powder was continued and further 7 days were lapsed, more hair growth was observed at the upper eyelid area at which inflammation was severe (Figs. 4D and E).

### [Treatment example 5: Treatment by combined liquid formulation of unidentified hair loss in dog]

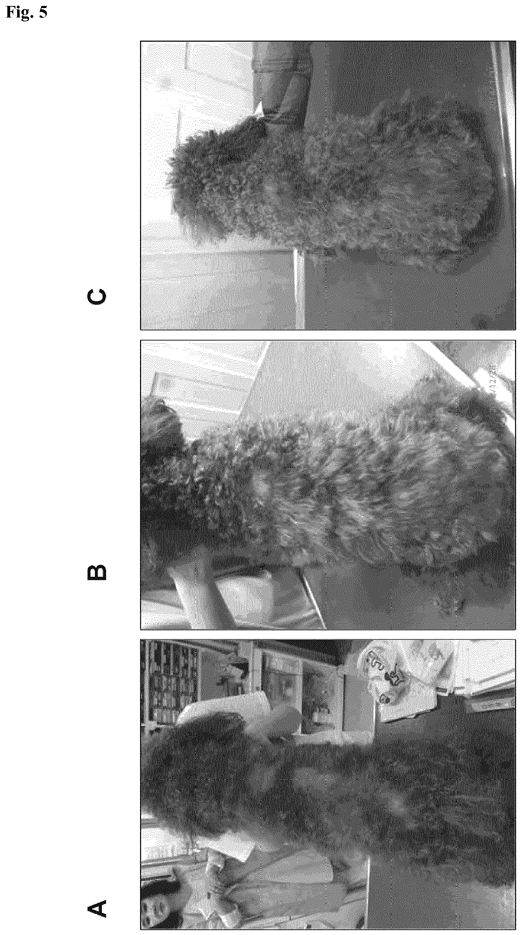

A female dog subjected to contraception with estimated 5-years-old having unidentified depilatory symptoms was treated by a combined liquid formulation. The state before the treatment is shown in Fig. 5A. Hair loss was observed in a wide range at the left back part, but no inflammation symptoms was observed. The combined liquid formulation was coated at around the hair lost part while patting once in 2 or 3 days. After 69 days were lapsed from starting the treatment, hair restoration was observed at the hair lost part (Fig. 5B). The treatment was further continued and 34 days were lapsed, the hair was so grown that the original hair lost part could not be identified whereby fur was improved (Fig. 5C).

## Claims

1. A composition for use in promoting hair restoration and/or hair growth comprising a sulfa agent and a chitosan agent.

2. The composition for use according to Claim 1, wherein the sulfa agent is selected from the group consisting of sulfamonomethoxine, acetyl sulfamethoxazole, salazosulfapyridine, sulfadiazine, silver sulfadiazine, sulfadimethoxine, sulfathiazole, sulfaphenazole, sulfamethoxazole, sulfamethoxypyridazine, sulfamethopyrazine, sulfametomidine, sulfamethizole, sulfamerazine, sulfisoxazole, sulfisomidine, sulfisomidine sodium, homosulfamine and a derivative thereof or wherein the sulfa agent is sulfamonomethoxine or a derivative thereof.

3. The composition for use according to any one of Claims 1 to 2, wherein the chitosan agent is a mixture of a high molecular weight chitosan and a low molecular weight chitosan or wherein the chitosan agent comprises chitin.

4. The composition for use according to any one of Claims 1 to 3, wherein the sulfa agent and the chitosan agent are mixed at a weight ratio of 20:1 to 1:20 or at a weight ratio of 5:1 to 1:5.

5. The composition for use according to any one of Claims 1 to 4, wherein a dosage form is powder or a liquid formulation.

6. The composition for use according to any one of Claims 1 to 5, wherein the hair restoration and/or hair growth is hair restoration and/or hair growth by alopecia selected from the group consisting of wound-derived alopecia, senile alopecia, stress-derived alopecia, male type alopecia, scarring alopecia, drug-derived alopecia and postpartum alopecia.

7. The composition for use according to any one of Claims 1 to 6, wherein an object is birds or mammals.

8. A sulfa agent and a chitosan agent for use in promoting hair restoration and/or hair growth, wherein the sulfa agent is applied separately from or simultaneously with a chitosan agent to an area of an object at which hair restoration and/or hair growth is required or wherein the chitosan agent is applied separately from or simultaneously with a sulfa agent to an area of an object at which hair restoration and/or hair growth is required.

9. A kit comprising a sulfa agent, a chitosan agent and optionally a dissipation container for use in promoting hair restoration and/or hair growth.

10. The sulfa agent and the chitosan agent for use according to Claim 8, wherein the sulfa agent is selected from the group consisting of sulfamonomethoxine, acetyl sulfamethoxazole, salazosulfapyridine, sulfadiazine, silver sulfadiazine, sulfadimethoxine, sulfathiazole, sulfaphenazole, sulfamethoxazole, sulfamethoxypyridazine, sulfamethopyrazine, sulfametomidine, sulfamethizole, sulfamerazine, sulfisoxazole, sulfisomidine, sulfisomidine sodium, homosulfamine and a derivative thereof or wherein the sulfa agent is sulfamonomethoxine or a derivative thereof.

11. The sulfa agent and the chitosan agent for use according to Claim 10, wherein the chitosan agent is a mixture of a high molecular weight chitosan and a low molecular weight chitosan or wherein the chitosan agent comprises chitin.

12. The sulfa agent and the chitosan agent for use according to any one of Claims 10 to 11, wherein the sulfa agent and the chitosan agent to be applied are at a weight ratio of 20:1 to 1:20 or at a weight ratio of 5:1 to 1:5.

13. The sulfa agent and the chitosan agent for use according to any one of Claims 10 to 12, wherein the sulfa agent and the chitosan agent to be applied are mixed in advance.

14. The sulfa agent and the chitosan agent for use according to any one of Claims 10 to 13, wherein a dosage form of the sulfa agent and the chitosan agent to be applied is powder or a liquid formulation.

15. The sulfa agent and the chitosan agent for use according to any one of Claims 10 to 14, wherein the application is carried out by using a dissipation container.

16. The sulfa agent and the chitosan agent for use according to any one of Claims 10 to 15, wherein the object is suffered from alopecia selected from the group consisting of wound-derived alopecia, senile alopecia, stress-derived alopecia, male type alopecia, scarring alopecia, drug-derived alopecia and postpartum alopecia.

17. The sulfa agent and the chitosan agent for use according to any one of Claims 10 to 16, wherein the object is birds or mammals.

## Patentansprüche

1. Zusammensetzung zur Verwendung bei der Förderung der Haarwiederherstellung und/oder des Haarwachstums, umfassend ein Sulfonamidagens und ein Chitosanagens.

2. Zusammensetzung zur Verwendung gemäß Anspruch 1, wobei das Sulfonamidagens ausgewählt ist aus der Gruppe, bestehend aus Sulfamonomethoxin, Acetylsulfamethoxazol, Salazosulfapyridin, Sulfadiazin, Silbersulfadiazin, Sulfadimethoxin, Sulfathiazol, Sulfaphenazol, Sulfamethoxazol, Sulfamethoxypyridazin, Sulfamethopyrazin, Sulfametomidin, Sulfamethizol, Sulfamerazin, Sulfisoxazol, Sulfisomidin, Sulfisomidin-Natrium, Homosulfamin und einem Derivat davon oder wobei das Sulfonamidagens Sulfamonomethoxin oder ein Derivat davon ist.

3. Zusammensetzung zur Verwendung gemäß einem der Ansprüche 1 bis 2, wobei das Chitosanagens eine Mischung aus einem Chitosan mit hohem Molekulargewicht und einem Chitosan mit niedrigem Molekulargewicht ist oder wobei das Chitosanagens Chitin umfasst.

4. Zusammensetzung zur Verwendung gemäß einem der Ansprüche 1 bis 3, wobei das Sulfonamidagens und das Chitosanagens in einem Gewichtsverhältnis von 20:1 bis 1:20 oder in einem Gewichtsverhältnis von 5:1 bis 1:5 gemischt sind.

5. Zusammensetzung zur Verwendung gemäß einem der Ansprüche 1 bis 4, wobei die Dosierungsform ein Pulver oder eine flüssige Formulierung ist.

6. Zusammensetzung zur Verwendung gemäß einem der Ansprüche 1 bis 5, wobei die Haarwiederherstellung und/oder das Haarwachstum eine Haarwiederherstellung und/oder ein Haarwachstum bei Alopezie ist, ausgewählt aus der Gruppe bestehend aus wundbedingter Alopezie, seniler Alopezie, stressbedingter Alopezie, Alopezie des Mannes, Narbenalopezie, arzneimittelbedingter Alopezie und postpartaler Alopezie.

7. Zusammensetzung zur Verwendung gemäß einem der Ansprüche 1 bis 6, wobei der Patient ein Vogel oder ein Säugetier ist.

8. Sulfonamidagens und Chitosanagens zur Verwendung bei der Förderung der Haarwiederherstellung und/oder des Haarwachstums, wobei das Sulfonamidagens getrennt von oder gleichzeitig mit einem Chitosanagens auf einen Bereich eines Patienten aufgetragen wird, an dem eine Haarwiederherstellung und/oder Haarwuchs erforderlich ist oder wobei das Chitosanagens getrennt von oder gleichzeitig mit einem Sulfonamidagens auf einen Bereich eines Patienten aufgebracht wird, an dem eine Haarwiederherstellung und/oder ein Haarwachstum erforderlich ist.

9. Kit, umfassend ein Sulfonamidagens, ein Chitosanagens und gegebenenfalls einen Dissipationsbehälter zur Verwendung bei der Förderung der Haarwiederherstellung und/oder des Haarwachstums.

10. Sulfonamidagens und Chitosanagens zur Verwendung gemäß Anspruch 8, wobei das Sulfonamidagens ausgewählt ist aus der Gruppe, bestehend aus Sulfamonomethoxin, Acetylsulfamethoxazol, Salazosulfapyridin, Sulfadiazin, Silbersulfadiazin, Sulfadimethoxin, Sulfathiazol, Sulfaphenazol, Sulfamethoxazol, Sulfamethoxypyridazin, Sulfamethopyrazin, Sulfametomidin, Sulfamethizol, Sulfamerazin, Sulfisoxazol, Sulfisomidin, Sulfisomidin-Natrium, Homosulfamin und einem Derivat davon oder wobei das Sulfonamidagens Sulfamonomethoxin oder ein Derivat davon ist.

11. Sulfonamidagens und Chitosanagens zur Verwendung gemäß Anspruch 10, wobei das Chitosanagens eine Mischung aus einem Chitosan mit hohem Molekulargewicht und einem Chitosan mit niedrigem Molekulargewicht ist oder wobei das Chitosanagens Chitin umfasst.

12. Sulfonamidagens und Chitosanagens zur Verwendung gemäß einem der Ansprüche 10 bis 11, wobei das Sulfonamidagens und das Chitosanagens, die angewendet werden sollen, in einem Gewichtsverhältnis von 20:1 bis 1:20 oder in einem Gewichtsverhältnis von 5:1 bis 1:5 vorliegen.

13. Sulfonamidagens und Chitosanagens zur Verwendung gemäß einem der Ansprüche 10 bis 12, wobei das Sulfonamidagens und das Chitosanagens, die angewendet werden sollen, im Voraus gemischt werden.

14. Sulfonamidagens und Chitosanagens zur Verwendung gemäß einem der Ansprüche 10 bis 13, wobei die Dosierungsform des Sulfonamidagens und des Chitosanagens, die angewendet werden sollen, ein Pulver oder eine flüssige Formulierung ist.

15. Sulfonamidagens und Chitosanagens zur Verwendung gemäß einem der Ansprüche 10 bis 14, wobei die Anwendung unter Verwendung eines Dissipationsbehälters erfolgt.

16. Sulfonamidagens und Chitosanagens zur Verwendung gemäß einem der Ansprüche 10 bis 15, wobei der Patient an einer Alopezie leidet, ausgewählt aus der Gruppe, bestehend aus wundbedingter Alopezie, seniler Alopezie, stressbedingter Alopezie, Alopezie des Mannes, Narbenalopezie, arzneimittelbedingter Alopezie und postpartaler Alopezie.

17. Sulfonamidagens und Chitosanagens zur Verwendung gemäß einem der Ansprüche 10 bis 16, wobei der Patient ein Vogel oder ein Säugetier ist.

## Revendications

1. Composition à utiliser pour favoriser la restauration de cheveux et/ou la pousse de cheveux comprenant un agent sulfa et un agent chitosane.

2. Composition à utiliser selon la revendication 1, dans laquelle l'agent sulfa est choisi parmi le groupe constitué de sulfamonométhoxine, acétyle sulfaméthoxazole, salazosulfapyridine, sulfadiazine, sulfadiazine d'argent, sulfadiméthoxine, sulfathiazole, sulfaphénazole, sulfaméthoxazole, sulfaméthoxypyridazine, sulfaméthopyrazine, sulfamétomidine, sulfaméthizole, sulfamérazine, sulfisoxazole, sulfisomidine, sulfisomidine sodium, homosulfamine et un dérivé de ceux-ci ou dans lequel l'agent sulfa est la sulfamonométhoxine ou un dérivé de celle-ci.

3. Composition à utiliser selon l'une quelconque des revendications 1 à 2, dans laquelle l'agent chitosane est un mélange d'un chitosane de poids moléculaire élevé et d'un chitosane de poids moléculaire faible ou dans laquelle l'agent de chitosane comprend de la chitine.

4. Composition à utiliser selon l'une quelconque des revendications 1 à 3, dans laquelle l'agent sulfa et l'agent chitosane sont mélangés selon un rapport en poids de 20:1 à 1:20 ou selon un rapport en poids de 5:1 à 1:5

5. Composition à utiliser selon l'une quelconque des revendications 1 à 4, dans laquelle une forme posologique est une poudre ou une formulation liquide.

6. Composition à utiliser selon l'une quelconque des revendications 1 à 5, dans laquelle la restauration de cheveux et/ou la pousse de cheveux est une restauration de cheveux et/ou une pousse de cheveux par alopécie choisie parmi le groupe comprenant une alopécie dérivée d'une plaie, alopécie sénile, alopécie dérivée d'un stress, alopécie de type masculin, alopécie cicatricielle, alopécie médicamenteuse et alopécie post-partum.

7. Composition à utiliser selon l'une quelconque des revendications 1 à 6, dans laquelle un objet est des oiseaux ou des mammifères.

8. Agent sulfa et agent chitosane à utiliser pour favoriser une restauration de cheveux et/ou une pousse de cheveux, l'agent sulfa étant appliqué séparément ou simultanément avec un agent chitosane sur une zone d'un objet sur lequel une restauration de cheveux et/ou une pousse de cheveux est requise ou dans lesquels l'agent chitosane est appliqué séparément ou simultanément avec un agent sulfa sur une zone d'un objet sur lequel une restauration de cheveux et/ou une pousse de cheveux est requise.

9. Kit comprenant un agent sulfa, un agent chitosane et facultativement un récipient à dissipation à utiliser pour favoriser une restauration de cheveux et/ou une pousse de cheveux.

10. Agent sulfa et agent chitosane à utiliser selon la revendication 8, dans lesquels l'agent sulfa est choisi parmi le groupe constitué de sulfamonométhoxine, acétyle sulfaméthoxazole, salazosulfapyridine, sulfadiazine, sulfadiazine d'argent, sulfadiméthoxine, sulfathiazole, sulfaphénazole, sulfaméthoxazole, sulfaméthoxypyridazine, sulfaméthopyrazine, sulfamétomidine, sulfaméthizole, sulfamérazine, sulfisoxazole, sulfisomidine, sulfisomidine sodium, homosulfamine et un dérivé de ceux-ci ou dans lequel l'agent sulfa est la sulfamonométhoxine ou un dérivé de celle-ci.

11. Agent sulfa et agent chitosane à utiliser selon la revendication 10, dans lesquels l'agent chitosane est un mélange d'un chitosane de poids moléculaire élevé et d'un chitosane de poids moléculaire faible ou dans lequel l'agent chitosane comprend la chitine.

12. Agent sulfa et agent chitosane à utiliser selon l'une quelconque des revendications 10 à 11, dans lesquels l'agent sulfa et l'agent chitosane à appliquer sont à un rapport en poids de 20:1 à 1:20 ou à un rapport en poids de 5:1 à 1:5.

13. Agent sulfa et agent chitosane à utiliser selon l'une quelconque des revendications 10 à 12, dans lesquels l'agent sulfa et l'agent chitosane à appliquer sont mélangés à l'avance.

14. Agent sulfa et agent chitosane à utiliser selon l'une quelconque des revendications 10 à 13, dans lesquels une forme posologique de l'agent sulfa et de l'agent chitosane à appliquer est une poudre ou une formulation liquide.

15. Agent sulfa et agent chitosane à utiliser selon l'une quelconque des revendications 10 à 14, dans lesquels l'application est réalisée en utilisant un récipient à dissipation.

16. Agent sulfa et agent chitosane à utiliser selon l'une quelconque des revendications 10 à 15, dans lesquels l'objet est atteint d'alopécie choisie parmi le groupe comprenant une alopécie dérivée d'une plaie, alopécie sénile, alopécie dérivée d'un stress, alopécie de type masculin, alopécie cicatricielle, alopécie médicamenteuse et alopécie post-partum.

17. Agent sulfa et agent chitosane à utiliser selon l'une quelconque des revendications 10 à 16, dans lesquels l'objet est des oiseaux ou des mammifères.
